# EUROPEAN PATENT APPLICATION

(11) **EP 2 058 299 A1**
(43) Date of publication of application: **13.05.2009**
(21) Application number: 07806343.5
(22) Date of filing: 30.08.2007
(51) Int. Cl.: C07C 267/00

(54) **METHOD FOR STABILIZING CARBODIIMIDE DERIVATIVE AND STABILIZED COMPOSITION THEREOF**

(30) Priority: 31.08.2006 JP 2006235364
(71) Applicant: Kaneka Corporation, Kita-ku Osaka-shi, Osaka 530-8288 (JP); Osaka Synthetic Chemical Laboratories, Inc., Nishinomiya-shi Hyogo 662-0934 (JP)
(72) Inventor: SENO, Kunihiko, Kume-gun Okayama 708-1527 (JP); OKUDA, Masao, Nishinomiya-shi Hyogo 662-0934 (JP); KAWASAKI, Hiroaki, Takasago-shi Hyogo 676-8688 (JP); MOROSHIMA, Tadashi, Takasago-shi Hyogo 676-8688 (JP); UEDA, Yasuyoshi, Takasago-shi Hyogo 676-8688 (JP)
(74) Representative: Hart-Davis, Jason
(86) International application number: PCT/JP2007/066866
(87) International publication number: WO 2008/026680

(57) **Abstract**

Disclosed is a method for stabilizing a carbodiimide derivative (1) which is a condensing agent useful for production of general synthetic chemical products. Also disclosed is a stabilized composition of the carbodiimide derivative. The method is **characterized in that** the carbodiimide derivative (1) is handled in an atmosphere wherein the concentration of molecular oxygen in the gas phase within a container is set at not more than 3% by volume and/or in the co-presence of at least one compound selected from the group consisting of antioxidants, N-oxyl compounds, sulfur compounds, amines and Lewis acids.

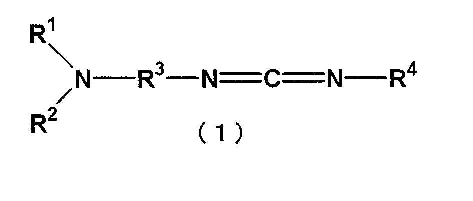

## Description

### TECHNICAL FIELD

The present invention relates to a method for stabilizing a carbodiimide derivative represented by general formula (1)

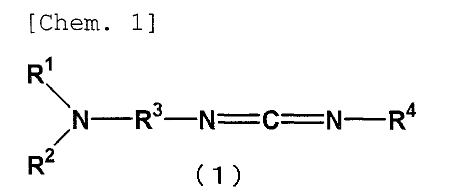

that is useful as a dehydration-condensation agent in organic synthesis reactions, and to a stabilized composition thereof.

### BACKGROUND ART

In general, a carbodiimide compound acts as a condensing agent when ester bonds and amides bond are formed. Because such a condensation reaction is used for not only for producing medicines and pesticides, but also for reactions to synthesize a variety of organic compounds, carbodiimide compounds have been widely used.

Particularly, the carbodiimide derivative represented by general formula (1) has the following characteristics: a) Urea which is formed as a by-product in the condensation reaction is easily removed from the reaction system because the urea is soluble in water under acidic conditions, and b) Racemization does not occur easily in a condensation reaction involving an optically active compound. Therefore, this carbodiimide derivative is known for demonstrating the outstanding advantages of high quality and low cost in obtaining a desired condensation product.

Carbodiimide derivatives are obtained, for example, by the desulfurization reaction of a corresponding thiourea derivative (patent documents 1 and 2). It is known, however, that in general carbodiimide derivatives without further treatment or modification are very unstable to water and heat (patent document 3), and it is difficult to put the derivatives on the market under moisture-proof, low temperature conditions. Therefore, as a reagent and commercial production item a carbodiimide derivative is normally provided as a solid salt of an acid, which can be handled with greater stability (patent document 4).

However, problems that arise in such a case include the following: (1) handling is poor because the acid salt is very hygroscopic, (2) the acid salt is bulkier than a liquid, which places a burden on transport, (3) the acid salt is difficult to handle like a liquid under sealed conditions, and a worker can be easily exposed thereto, and (4) when the acid salt is used in an anhydrous condensation reaction, the inconvenience of dissociating the acid salt is sometimes required. These complex operability problems have been extremely disadvantageous, especially in the use of carbodiimide derivative acid salts on a commercial scale. Therefore, the establishment of a method for handling carbodiimide compounds stably on a commercial scale without conversion to an acid salt has been earnestly sought.
[Patent document 1] Japanese Patent Application Laid-open No. H4-77464
[Patent document 2] Japanese Patent Application Laid-open No. H8-198836
[Patent document 3] Japanese Patent Application Laid-open No. H8-277254
[Patent document 4] Japanese Patent Application Laid-open No. H9-110818

### DISCLOSURE OF THE INVENTION

With the foregoing in view, an object of the present invention is to provide a method for inhibiting degradation of carbodiimide derivative (1) so that it can be handled stably on a commercial scale, and a composition whereby carbodiimide derivative (1) is stabilized.

The inventors discovered that carbodiimide derivative (1) can be maintained with extreme stability by holding it in an atmosphere with a specific molecular oxygen concentration, and this can impart sufficient durability for handling thereof on a commercial scale. In addition, the inventors discovered a method wherein at least one compound selected from the group consisting of an antioxidant, N-oxyl compound, sulfur compound, amine, and Lewis acid is present together with carbodiimide derivative (1) as a method that achieves that object even more simply and efficiently, thus completing the present invention.

More specifically, the present invention relates to a method for stabilizing a carbodiimide derivative wherein carbodiimide derivative (1) is handled: in an atmosphere having a molecular oxygen concentration of 3vol% or less in the gas phase in the container; and/or in the co-presence of at least one compound selected from the group consisting of an antioxidant, N-oxyl compound, sulfur compound, amine, and Lewis acid; a stabilized composition in accordance with that method, and a packaged article thereof.
The present invention is described in detail below.

First, carbodiimide derivative (1) used in the present invention is described.
In the carbodiimide derivative used in the present invention represented by general formula (1),

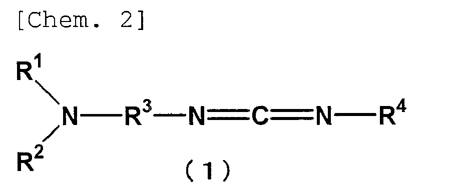

R¹, R², and R⁴ may be the same or different, and represent a hydrogen atom, an optionally substituted C₁₋₁₀ alkyl group, or optionally substituted C₇₋₁₅ aralkyl group. The C₁₋₁₀ alkyl group is not particularly limited herein, and can be straight-chain, branched, cyclic or non-cyclic. Examples thereof include a methyl, ethyl, n-propyl, isopropyl, n-butyl, isobutyl, t-butyl, n-pentyl, n-hexyl, cyclohexyl, n-heptyl, n-octyl, n-nonyl, or n-decyl group and the like. Examples of the C₇₋₁₅ aralkyl group include a benzyl, phenethyl, phenylpropyl, phenylbutyl, diphenylmethyl, or naphthylpentyl group and the like.

For R¹ and R², C₁₋₁₀ alkyl groups are preferred, methyl, ethyl, n-propyl, and n-butyl groups are more preferred, and methyl groups are particularly preferred. For R⁴, a C₁₋₁₀ alkyl group is preferred, a methyl, ethyl, n-propyl or n-butyl is more preferred, and an ethyl group is particularly preferred.

R³ represents an optionally substituted C₁₋₁₀ alkylene group, optionally substituted C₂₋₁₀ alkenylene group, optionally substituted C₂₋₁₀ alkynylene group, or optionally substituted bivalent aromatic group. The aforementioned alkylene, alkenylene, and alkynylene group is not particularly limited herein, and can be straight-chain, branched, cyclic or non-cyclic. Examples of the alkylene group include a methylene, ethylene, propylene, tetramethylene, or hexamethylene group, and the like; examples of the alkenylene group include a vinylene, propenylene, or butadienylene group and the like; and examples of the alkynylene group include an ethynylene, propynylene, or butynylene group, and the like. Examples of the bivalent aromatic group include a phenylene, naphthylene, biphenylene, anthracenedyl, pyridinedyl, thiophenedyl, fluorophenylene, chlorophenylene, methylphenylene, silylphenylene, hydroxyphenylene, or aminophenylene group and the like. Among the above, a C₁₋₁₀ alkylene group is preferred, an ethylene, propylene, or tetramethylene group is more preferred, and a propylene group is even more preferred as R³.

The above "substituent" is not particularly limited herein, and examples thereof include a halogen atom, hydroxyl group, amino group, carboxyl group, ether group and the like.

Carbodiimide derivative (1) prepared by various methods can be used, and the method for obtaining the same is not particularly limited herein. Following the methods described, for example in patent document 1 or patent document 2, the carbodiimide derivative obtained by desulfurization of the thiourea derivative represented by general formula (2)

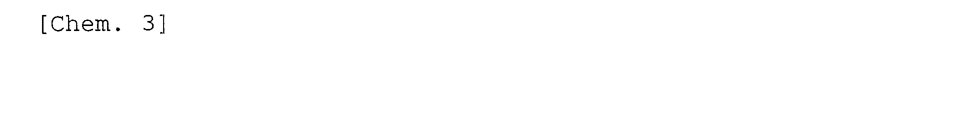

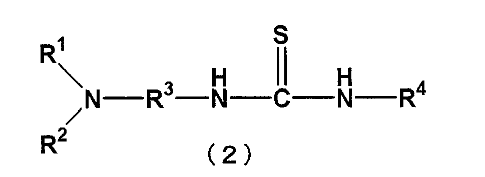

can be used. Thiourea derivative (2) prepared by various methods can be used and, following the publicly known technique described in patent document 2, for example, the thiourea derivative obtained by reacting the isothiocyanate derivative represented by general formula (3);

R⁴ - N = C = S (3)

with the amine derivative represented by general formula (4)

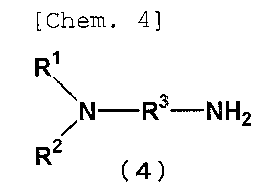

can be used. R¹, R², R³, and R⁴ in general formulas (2), (3), and (4) above are equivalent to R¹, R², R³, and R⁴ in general formula (1) above.

Carbodiimide derivative (1) obtained by desulfurization of thiourea derivative (2) can be purified as needed. The purification method is not particularly limited herein, and purification by distillation and various chromatography methods can be used. For example, following the method described in patent document 4, it can be converted to a salt, precipitated as crystals, and neutralized, or it can be purified by distillation and various chromatography methods, etc., and can be used after conversion to a salt, crystallization, and neutralization. In addition, the commercially available hydrochloride of carbodiimide derivative (1) can be neutralized and used.

The mode and method of distillation is not particularly limited herein, and examples include simple distillation, rectification, thin film distillation, etc., either as batch or continuous distillation. The distillation conditions are not particularly limited herein, and as described in patent document 3, in consideration of the stability of this compound, either vacuum distillation that can be performed at a lower temperature range or azeotropic distillation with a substance having a similar boiling point is preferred.

Neutralization of the ammonium salt that was obtained by conversion to a salt and precipitation as crystals can be performed by a method treating with a base in a mixed solvent consisting of a water-immiscible organic solvent and water, or a method treating with a base in water, and then extracting in a water-immiscible organic solvent is performed.

The water-immiscible organic solvent is not particularly limited herein, and examples include pentane, hexane, heptane, octane and other aliphatic hydrocarbon solvents; benzene, toluene, xylene and other aromatic hydrocarbon solvents; methylene chloride, chlorobenzene, chloroform, 1,1,1-trichloroethane, and other halogenated solvents; methyl tert-butyl ether, dibutyl ether, and other ether solvents; ethyl acetate, n-propyl acetate, isopropyl acetate, n-butyl acetate, tert-butyl acetate, and other ester solvents, methyl isobutyl ketone and other ketone solvents; and tert-butyl alcohol and other alcohol solvents. These organic solvents can be used alone or as a mixture thereof. Furthermore, as needed, a different water-miscible organic solvent, for example, acetone, acetonitrile, methanol, etc., can be mixed therewith in a range that does not adversely affect the action thereof. The sequence of mixing is not particularly limited herein.

The base is not particularly limited herein provided it can neutralize the acid component. Examples include sodium hydroxide, lithium hydroxide, potassium hydroxide and hydroxides of other alkali metals; magnesium hydroxide, calcium hydroxide, and hydroxides of other alkaline earth metals; sodium carbonate, potassium carbonate, lithium carbonate and carbonates of other alkali metals; sodium bicarbonate, potassium bicarbonate, lithium bicarbonate and bicarbonates of other alkali metals; sodium methoxide, sodium ethoxide, lithium isopropoxide, potassium t-butoxide, and other alkoxides of alkali metals; sodium acetate, potassium acetate, lithium acetate, and other alkali metal-organic acid salts. Among the above, inorganic salts are preferred because they are inexpensive and easy to handle, and the hydroxides of alkali metals and the carbonates of alkali metals are more preferred. The base can be used in a liquid state such as an aqueous solution, etc., or it can be used unaltered as a solid.

The procedure of the neutralization treatment is not particularly limited herein and, for example, after an aqueous solution of the base is first brought into contact with the ammonium salt of carbodiimide derivative (1) and neutralization is carried out, extraction with a water-immiscible organic solvent can be performed, or neutralization can be carried out while a water-immiscible organic solvent added is also present.

The neutralization temperature is not particularly limited herein, and neutralization can be performed at or below the boiling point of the used solvents, but at a temperature wherein the solvents do not solidify. In consideration of the stability of carbodiimide derivative (1), carrying out the neutralization treatment at a lower temperature is preferred. The neutralization treatment concentration depends on the solubility of carbodiimide derivative (1) in the used solvents, but it is not particularly limited herein, and normally the neutralization treatment can be carried out at a concentration of 1 to 70%.

After treatment with the base, carbodiimide derivative (1) is obtained as a solution in the water-immiscible organic solvent, and the solvent can be removed as needed. The method for removing the solvent is not particularly limited herein and, for example, the solvent can be removed by enrichment under normal pressure or reduced pressure conditions, and when removal at a lower temperature is desired, the solvent can be suitably removed using a device for thin layer distillation, which has a short thermal history, and depending on the type of solvent, freeze-drying can also be used.

This neutralization treatment is very useful for obtaining carbodiimide derivative (1) stably, and can be used when obtaining carbodiimide derivative (1) from a salt thereof other than the ones noted above.

Next, the method for handling carbodiimide derivative (1) in the present invention is specifically described.

Carbodiimide derivative (1) used in the present invention is unstable to water, and therefore very little moisture content is preferred. More specifically, the moisture content is preferably 5wt% or less, more preferably 1wt% or less, and even more preferably 0.2wt% or less. Enrichment, distillation, as well as the use of dehydrating agents, etc., can be noted as methods for reducing the moisture content.

Because carbodiimide derivative (1) is unstable to primary amines, when carbodiimide derivative (1) obtained by desulfurization of the aforementioned thiourea derivative (2) is used, preferably there is less contamination by amine derivative (4), which is used as a raw material thereof. The content of amine derivative (4) is preferably 1wt% or less, more preferably 0.5wt% or less, and even more preferably 0.1wt% or less.

First the molecular oxygen concentration in the container to be used when handling carbodiimide derivative (1) is explained.
In the present method, the term "container" can refer to a container filled with carbodiimide derivative (1) for the purpose of reacting, storing, stockpiling, or transporting the same. When handling carbodiimide derivative (1) on a commercial scale, the container may take the form of a storage vessel surrounded by a wall, provided the oxygen concentration can be maintained at the predetermined value therein. The capacity of the aforementioned container is not particularly limited herein, but for the advantage of the present invention to be exhibited even more, the capacity is suitable for handling on a commercial scale, preferably 0.5 L or more, more preferably 5 L or more, even more preferably 50 L or more, and most preferably 200 L or more. However, preferably the capacity is no more than 25000 L.

Generally, when handling a flammable substance the required molecular oxygen concentration is 12.5vol% or less from the standpoint of fire prevention, but under such conditions carbodiimide derivative (1) cannot be stabilized on a commercial scale. To achieve the object of the present invention it is necessary to restrict the molecular oxygen concentration even more stringently.

As its upper limit, in the present invention the range of the molecular oxygen concentration in the gas phase that contacts with carbodiimide derivative (1) during handling of the same is 3vol% or less, preferably 2vol% or less, and more preferably, 1vol% or less. On the other hand, the lower the molecular oxygen concentration is maintained, the more the stabilizing effect increases, so the lower limit thereof is not limited herein. In actual handling on a commercial scale, however, it is extremely difficult to prevent contamination by trace amounts of oxygen from the outside. In consideration of the fact that contamination by a very small amount of oxygen is essentially unavoidable, and also the fact that the stabilizing effect obtained by maintaining a low oxygen concentration is sufficient provided the required level of stabilization from a commercial standpoint is attained, the lower level of molecular oxygen concentration, for example, is preferably 0.01vol% or more, more preferably 0.05vol% or more, and even more preferably 0.1vol% or more.
In the co-presence of the antioxidant, etc., described below, the oxygen concentration is not necessarily limited to 3vol% or less.

The gas phase volume in the container after filling with carbodiimide derivative (1) is not particularly limited herein provided the molecular oxygen concentration is within the above range, and, for example, carbodiimide derivative (1) is preferably be loaded at a rate of 30vol% or more, more preferably 50vol% or more, and even more preferably 80vol% or more.

It is preferable for the molecular oxygen concentration dissolved in carbodiimide derivative (1) to be kept low and, for example, it is preferably 10 ppm or less, more preferably 5 ppm or less, and even more preferably 3 ppm or less.

The method for reducing the concentration of molecular oxygen can be any method without regard to the gas phase or dissolved amount, and a method should be selected that is suitable for conditions at the time of handling. For example, a method wherein the molecular oxygen is removed to outside the system together with the gas of the gas phase or dissolved gas itself by maintaining a vacuum, and a method wherein the gas phase is replaced by a gas that is inert with respect to carbodiimide derivative (1) can be noted.

The replacement method is not particularly limited herein and, for example, an inert gas can be introduced until the gas phase in contact with carbodiimide derivative (1) reaches the desired molecular oxygen concentration, or insertion of the inert gas can be performed repeatedly after the pressure inside the container is once reduced. Examples of gases that are inert with respect to carbodiimide derivative (1) include helium, neon, argon, and other rare gases, or nitrogen, carbon dioxide, and the like. From an industrial standpoint, nitrogen and argon are preferred because they are low in cost and easy to obtain.

As examples of other methods, the molecular oxygen concentration of the gas phase or the dissolved molecular oxygen concentration can be reduced by a commercial oxygen absorber such as AGELESS^{™} (Mitsubishi Gas Chemical Co., Inc.), etc. In addition, using a method whereby the container is sealed under conditions such that the pressure in the container is kept higher than the outside pressure, or using methods that utilize a container with increased sealing tightness due to multiple packaging, or a container having a material such as an aluminum laminate that does not allow the passage of oxygen is effective in preventing contamination by molecular oxygen from the outside and can achieve the object more efficiently. Furthermore, the method wherein a container that contains carbodiimide derivative (1) is placed inside another container (multiple packaging), an oxygen absorber is placed outside the container containing carbodiimide derivative (1), and both are then packed in another container, etc., is effective, but the method for introducing an oxygen absorber is not limited thereto.

Next, the stabilizing method wherein carbodiimide derivative (1) is handled in the co-presence of at least one compound selected from the group consisting of an antioxidant, N-oxyl compound, sulfur compound, amine, and Lewis acid is described.

The term "in the co-presence of" is defined as being present together in the container used for handling carbodiimide derivative (1).

The aforementioned antioxidant is not particularly limited herein, but primary antioxidants, secondary antioxidants, and metal deactivators can be noted, and specifically, the following compounds can be listed: Quinone series primary antioxidants such as hydroquinone, methoxy hydroquinone, benzoquinone, p-tert-butyl catechol, chloranil, 2-tert-butyl hydroquinone, 2,5-di-tert-butyl hydroquinone, 2-tert-butyl methoxy hydroquinone, 2,5-di-tert-amino hydroquinone, and polymers carrying the same;

Alkylphenol series primary antioxidants such as 2,6-di-tert-butyl phenol, 2-tert-butyl-4,6-dimethyl phenol, 2,6-di-tert-butyl-4-methyl phenol, 2,6-di-tert-butyl-4-ethyl phenol, 2,6-di-tert-butyl-4-hydroxymethyl phenol, 2,4,6-tri-tert-butyl phenol, 3,5-di-tert-butyl-4-hydroxy anisole, 3,5-dialkyl-4-hydroxybenzyl ether, 4,4'-methylene bis(2,6-dimethyl phenol), 2,2'-methlene bis(4-methyl-6-tert-butyl phenol), 4,4'-thio bis(6-tert-butyl-m-cresol), and polymers carrying the same;

Amine series primary antioxidants (secondary amines having an N,N-diaryl structure) such as diphenyl amine, alkylated diphenyl amine, N,N'-diphenyl-p-phenylene diamine, 6-ethoxy-2,2,4-trimethyl-1,2-dihydroquinoline, N-phenyl-N'-isopropyl-p-phenylene diamine, 2,2,4-trimethyl-1,2-dihydroquinoline polymer, aldol-α-naphthyl amine, N-phenyl-β-naphthyl amine, 4,4'-dioctyl diphenyl amine, and polymers carrying the same;

Copper dithiocarbamate series primary antioxidants such as copper dimethyl dithiocarbomate, copper diethyl dithiocarbomate, copper dipropyl dithiocarbamate, copper dibutyl dithiocarbamate, copper ethylene dithiocarbamate, copper tetramethylene dithiocarbamate, copper pentamethylene dithiocarbamate, copper hexamethylene dithiocarbamate, copper oxydiethylene dithiocarbmate, and polymers carrying the same;

Sulfur series secondary antioxidants such as dilauryl-3,3'-thio dipropionate, di-tridecyl-3,3'-thio dipropionate, dimyristyl-3,3'-thio dipropionate, distearyl-3,3'-thio dipropionate, tetrakis [methylene-3-(laurylthio) propionate] methane, distearyl-3,3'-methyl-3,3'-thio dipropionate, laurylstearyl-3,3'-thio dipropionate, bis[2-methyl-4-(3-n-alkyl thiopropionyloxy-5-tert-butylphenyl) sulfide, β-laurylthio propionate, 2-mercapto benzimidazole, 2-mercapto-5-methyl benzimidazole, and polymers carrying the same;

Phosphorus series secondary antioxidants such as triethyl phosphite, tris(isodecyl) phosphite, phenyl diisooctyl phosphite, diphenyl isodecyl phosphite, triphenyl phosphite, tris(2,4-di-tert butylphenyl) phosphite, tris(biphenyl) phosphite, phenyl-bisphenol A-pentaerythritol diphosphite, 1,3-bis(diphenoxy-phosphonyloxy) benzene, and polymers carrying the same;

And metal deactivators such as ethylenediamine tetraacetic acid, hydroxyethyl ethylenediamine triacetic acid, dihydroxyethyl ethylenediamine diacetic acid, 1,3-propane diamine tetraacetic acid, diethylene triamine pentaacetic acid, triethylene tetramine hexaacetic acid, nitrilo-triacetic acid, sodium gluconate, hydroxyethyl iminodiacetic acid, citric acid, tartaric acid, and polymers carrying the same.

Among the above, alkylphenol series primary antioxidants, amine series primary antioxidant, phosphorus series secondary antioxidants, and metal deactivators are most suitably used, preferably 2,6-di-tert-butyl phenol, 2-tert-butyl-4,6-dimethyl phenol, 2,6-di-tert-butyl-4-methyl phenol, 2,6-di-tert-butyl-4-ethyl phenol, 2,6-di-tert-butyl-4-hydroxymethyl phenol, 2,4,6-tri-tert-butyl phenol, diphenyl amine, alkylated diphenyl amine, N,N'-diphenyl-p-phenylene diamine, N-phenyl-N'-isopropyl-p-phenylene diamine, 4,4'-dioctyl diphenyl amine, triethyl phosphite, tris(isodecyl) phosphite, hydroxyethyl ethylenediamine triacetic acid, dihydroxyethyl ethylenediamine diacetic acid, hydroxyethyl imino diacetic acid, and citric acid; and more preferably 2,6-di-tert-butyl-4-methyl phenol, diphenyl amine, triethyl phosphite, and citric acid.

The aforementioned N-oxyl compound is not particularly limited herein, and examples include cyclohexane-1-spiro-2'-(4'-oxoimidazolidine-1'-oxyl)-5'-spiro-1"-cyclohexane, cyclohexane-1-spiro-2'-(4'-oxoimidazolidine-1'-hydroxyl)-5'-spiro-1"-cyclohexane, cyclohexane-1-spiro-2'-(4'-oxoimidazolidine-1'-methoxy)-5'-spiro-1"-cyclohexane, 2,2,6,6-tetramethyl piperidine-N-oxyl, 4-hydroxy-2,2,6,6-tetramethyl piperidine-N-oxyl, 4-acetyloxy-2,2,6,6-tetramethyl piperidine-N-oxyl, 4-acryloyloxy-2,2,6,6-tetramethyl piperidine-N-oxyl, 4-methacryloyloxy-2,2,6,6-tetramethyl piperidine-N-oxyl, 4-benzoyloxy-2,2,6,6-tetramethyl piperidine-N-oxyl, 4-methoxy-2,2,6,6-tetramethyl piperazine-N-oxyl, N-hydroxy succinimide, 1-hydroxy benzotriazole, 3-hydroxy-4-oxo-3,4-dihydro-1,2,3,-benzotriazine, 1-hydroxy-2-oxo-indoline, 3-hydroxy-4-oxo-3,4-dihydroquinazoline, 1-hydroxy-2(1H)-pyridone, etc. Among the above, 4-hydroxy-2,2,6,6-tetramethyl piperidine-N-oxyl, N-hydroxy succinimide, and 1-hydroxy benzotriazole are preferred.

The aforementioned sulfur compound is not particularly limited herein, and examples include sulfur, lithium sulfide, sodium sulfide, potassium sulfide, ammonium sulfide, carbon disulfide, hydrogen sulfide, organosulfide compounds, etc. Among the above, sulfur and carbon disulfide are preferred.

The above amine compound is not particularly limited herein provided it is not a primary amine. For example, the amine represented by general formula (5)

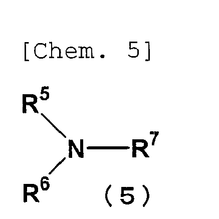

can be noted. In general formula (5) above, R⁵ and R⁶ represent either the same or different optionally substituted C₁₋₁₀ alkyl groups, optionally substituted C₆₋₁₅ aryl groups, or optionally substituted C₇₋₁₅ aralkyl groups. R⁷ represents a hydrogen, optionally substituted C₁₋₁₀ alkyl group, optionally substituted C₆₋₁₅ aryl group, or optionally substituted C₇₋₁₅ aralkyl group. The C₁₋₁₀ alkyl group is not particularly limited herein and can be straight-chain, branched, cyclic, or non-cyclic. Examples include a methyl, ethyl, n-propyl, isopropyl, n-butyl, isobutyl, t-butyl, n-pentyl, n-hexyl, cyclohexyl, n-heptyl, n-octyl, n-nonyl, or n-decyl group, and the like. Examples of the C₆₋₁₅ aryl group include a phenyl or naphthyl group and the like. Examples of the C₇₋₁₅ aralkyl group include a benzyl, phenethyl, phenylpropyl, phenylbutyl, diphenylmethyl, or naphthylpentyl group and the like. However, a secondary amine having an N,N-diaryl amine structure in which two substituents selected from R⁵, R⁶ and R⁷ constitute aryl groups is not included among the above amine compounds as defined herein.
Among the above, a C₁₋₁₀ alkyl group is preferred as R⁵, R⁶, and R⁷, a methyl, ethyl, n-propyl, or n-butyl group is more preferred, and an ethyl group is even more preferred. Among the above amine compounds, triethylamine is preferred.

The aforementioned Lewis acid is not particularly limited herein, and examples include a chloride, bromide, iodide, lower alkyl compound, complex and the like having as the central atom thereof aluminum, tin, zirconium, magnesium, boron, titanium, zinc, silicon, iron, germanium, antimony, hafnium, bismuth, scandium, ytterbium, manganese, cobalt, nickel, copper, gallium, samarium, cerium, vanadium, tungsten and the like. A chloride, bromide, iodide, lower alkyl compound and complex having boron as the central atom thereof is preferred, and a boron trifluoride ether complex is particularly preferred.

The amount to be added of the aforementioned "at least one compound selected from the group consisting of an antioxidant, N-oxyl compound, sulfur compound, amine, and Lewis acid" differs depending on the type of compound and cannot be generally stated. However, when the use of carbodiimide derivative (1) as a condensing agent, operability, and cost-effectiveness are all taken into consideration, the presence of a large amount of impurities is undesirable. Therefore, the upper limit is preferably 20wt% or less, more preferably 10wt% or less, and even more preferably 5wt% or less with respect to carbodiimide derivative (1). The lower limit is preferably 0.0001wt% or more, and more preferably 0.0005wt% or more, with respect to carbodiimide derivative (1).

The method using these additives can be combined with the aforementioned method for controlling molecular oxygen at a low concentration to increase the stabilization of carbodiimide derivative (1) even more. This is particularly effective as a countermeasure against the risk of molecular oxygen contamination when cases of handling on a commercial scale such as long term storage and stockpiling is considered. Industrially speaking, the stabilized composition comprising carbodiimide derivative (1) and at least one compound selected from the group consisting of an antioxidant, N-oxyl compound, sulfur compound, amine, and Lewis acid extremely useful because carbodiimide derivative (1) can be handled relatively stably.

The temperature for handling carbodiimide derivative (1) and the composition thereof stabilized by the above method depends on the necessary conditions at the time of handling, and is not particularly limited herein. However, 85°C or lower is preferred, 50°C or lower is more preferred, and 30°C or lower is particularly preferred. On the other hand, a temperature so low that it requires special refrigeration equipment is not necessary for handling such as storage and stockpiling, and a lower limit of -50°C or higher is preferred, -30°C or higher is more preferred, -20°C or higher is even more preferred, and -10°C or higher is particularly preferred.

The moisture content of the atmosphere for handling carbodiimide derivative (1) and the composition thereof stabilized by the above method depends on the conditions at the time of handling, but a low moisture content is more preferred. As an upper limit, a relative humidity in the container of 80% or lower is preferred, 70% or lower is more preferred, and 60% or lower is particularly preferred.

The above stabilizing method also displays a color stabilizing effect and can be used to inhibit discoloration due to decomposition. In the composition stabilized by the above method a discoloration level of 300 Hazen units or less, preferably 200 Hazen units or less, and more preferably 100 Hazen units or less can be attained. In this case the Hazen color scale is measured in accordance with the method of JIS K0071-1.

The term "handling" in the present invention refers to handling carbodiimide derivative (1) in a single operation such as filling, packaging, stockpiling, storing, or transporting. When the scale of industrial production is considered, a general estimate is, for example, that carbodiimide derivative (1) can preferably be stored for 3 months or longer, more preferably 4 months or longer, and most preferably 6 months or longer with a decomposition rate of 10% or less and at a storage temperature attainable by general facilities, for example 5°C or higher. This estimate takes into consideration market distribution conditions such as the stocking period thereof as a product, or the retention of surplus inventory that occurs when the product is used. The materials of the equipment and containers used during handling are not particularly limited herein, and generally materials for manufacturing equipment such as glass linings, SUS, and the like and materials for containers such as chemical drums, steel drums, and the like are acceptable. Containers of materials that do not allow the passage of oxygen such as aluminum laminate and polyethylene bags, etc., with low oxygen permeability are acceptable. A method suited to the conditions during handling should be selected from among the aforementioned methods to reduce the concentration of molecular oxygen at that time. More specifically, when filling a container, etc., if the oxygen concentration can be kept low with a method such as appropriate sealing so that contamination of oxygen from the outside can be blocked, no special measures or equipment other than those described above is required, and commercially available drums and the like can be used. As needed, however, filling under positive pressure such that oxygen contamination is blocked, packaging together with an oxygen absorber, or packaging that involves multiple packaging using the above container is preferred because carbodiimide (1) can be kept even more stable.
In the present invention the term "carbodiimide derivative (1) packaged article" refers to the container itself that contains carbodiimide derivative (1) and also to a packaged article wherein the container containing carbodiimide derivative (1) is packaged according to the aforementioned method. The packaged article of the present invention, characterized by containing a carbodiimide derivative (1) and having a molecular oxygen concentration of 3vol% or less in the gas phase in the container; and/or containing at least one compound selected from the group consisting of an antioxidant, N-oxyl compound, sulfur compound, amine, and Lewis acid, is extremely useful for industrial stockpiling, storage, transport, etc., because carbodiimide derivative (1) can be maintained with stability therein.

According to the present invention, carbodiimide derivative (1) can be handled stably while the degradation thereof is inhibited.

### BEST MODE FOR CARRYING OUT THE INVENTION

The present invention is explained in even greater detail by the following examples, but is by no means limited thereto.

In the examples the content of carbodiimide derivative was measured by gas chromatography under the following conditions.
Column packing: Silicone SE-30 Chromosorb WAW DMCS 5% 80/100 mesh
Column size: 3 mm × 2 m
Column temperature: 70°C to 270°C
Inlet temperature: 250°C
Detector temperature: 300°C
Carrier gas: nitrogen
Detector: FID

Hazen color units were measured in accordance with the method of JIS K0071-1.

The time required to reach a degradation rate of 10% was measured as T90, and calculated by the Arrhenius equation generally used for calculating degradation time based on accelerated testing. As recommended in YOSHIOKA, Sumie, Stability of Pharmaceutical Products, published by Nankodo Co., Ltd. (1995), for example, the lowest value (10kcal/mol) in the observed range for normal chemical degradation was used for the activation energy at that time.

### (Example 1)

In a glove box the oxygen concentration was adjusted to either 20vol%, 10vol%, 3vol%, 1vol%, or 0.1vol% with nitrogen, and under each atmospheric condition 300 g of 1-ethyl-3-(3-dimethylaminopropyl) carbodiimide (moisture content 0.05wt%, here and in subsequent examples) was placed in a 500 mL test container, and the containers were sealed. The test containers were stored for 14 days at an external temperature of 40°C and 75% RH. From each content value measured at days 3, 7, and 14, the degradation rate per day was determined as the degradation rate. Table 1 shows both the Hazen units and T90 value at 5°C. The T90 value at 5°C was obtained by converting the results from storage at 40°C for 14 days to a storage period at 5°C using the Arrhenius equation.

**[Table 1]**

| Oxygen concentration (% by volume) | Degradation rate (%/day) | Color in Hazen unit | T90 value at 5°C (month) |
|---|---|---|---|
| 0.1 | 0.2 | 10 | 14 |
| 1 | 0.3 | 30 | 9 |
| 3 | 0.4 | 40 | 6 |
| 10 | 0.7 | 200 | 4 |
| 20 | 1.0 | 300 | 2 |

### (Example 2)

In a glove box the oxygen concentration was adjusted to 0.1vol% with nitrogen, and 300 g of 1-ethyl-3-(3-dimethylaminopropyl) carbodiimide was placed in each 500 mL test container. To the above 2,6-di-tert-butyl-4-methylphenol (BHT) was added at either 0wt%, 0.01wt%, or 1wt% with respect to the 1-ethyl-3-(3-dimethylaminopropyl) carbodiimide, and the containers were sealed. After the containers were stored for 14 days at an external temperature of 40°C and 75% RH, the content was measured. Table 2 shows the results together with the T90 value at 5°C.

**[Table 2]**

| BHT addition amount (% by weight) | Content after storage (%) | T90 value at 5°C (month) |
|---|---|---|
| 0 | 97 | 14 |
| 0.01 | 98 | 18 |
| 1 | 99 | 36 |

### (Example 3)

In a glove box the oxygen concentration was adjusted to either 20vol% or 1vol% with nitrogen, and 300 g of 1-ethyl-3-(3-dimethylaminopropyl) carbodiimide was placed in each 500 mL test container, and the containers were sealed. After the containers were stored for 20 hours at an external temperature of 85°C, the content was measured.
Table 3 shows the results.

**[Table 3]**

| Oxygen concentration (% by volume) | Content after storage (%) |
|---|---|
| 1 | 97 |
| 20 | 55 |

### (Example 4)

At an oxygen concentration of 20vol%, 300 g of 1-ethyl-3-(3-dimethylaminopropyl) carbodiimide was placed in 500 mL test containers. To the above 2,6-di-tert-butyl-4-methylphenol (BHT) was added at either 0wt%, 0.1wt%, or 2wt% with respect to the 1-ethyl-3-(3-dimethylaminopropyl) carbodiimide, and the containers were sealed. After the containers were stored for 20 hours at an external temperature of 85°C, the content was measured. Table 4 shows the results.

**[Table 4]**

| BHT addition amount (% by weight) | Content after storage (%) |
|---|---|
| 0 | 55 |
| 0.1 | 99 |
| 2 | 100 |

### (Example 5)

At an oxygen concentration of 20vol%, 200 g of 1-ethyl-3-(3-dimethylaminopropyl) carbodiimide was placed in a series of 500 mL test containers. The additives listed below were added to each container at 2wt% with respect to the 1-ethyl-3-(3-dimethylaminopropyl) carbodiimide, and the containers were sealed. After the containers were stored for 20 hours at an external temperature of 85°C, the content was measured. A sample to which no additive was added was prepared as a control and handled in the same manner. Table 5 shows the results.

**[Table 5]**

| Additive | Content after storage (%) |
|---|---|
| Absent (Control) | 55 |
| 2,6-di-tert-butyl-4-methylphenol | 100 |
| diphenyl amine | 99 |
| triethyl phosphite | 75 |
| citric acid | 68 |
| 4-hydroxy-2,2,6,6-tetramethyl piperidine-N-oxyl | 83 |
| N-hydroxy succinimide | 82 |
| 1-hydroxy benzotriazole | 70 |
| sulfur | 82 |
| carbon disulfide | 75 |
| triethylamine | 75 |
| boron trifluoride ether complex | 69 |

## Claims

1. A method for stabilizing a carbodiimide derivative,
**characterized in that** a carbodiimide derivative represented by general formula (1) 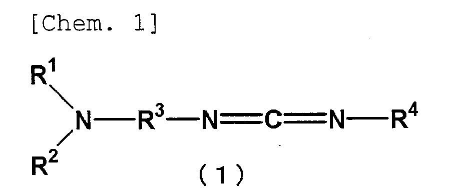 (wherein R¹, R², and R⁴ may be the same or different, and represent a hydrogen atom, an optionally substituted C₁₋₁₀ alkyl group, or optionally substituted C₇₋₁₅ aralkyl group; and R³ represents an optionally substituted C₁₋₁₀ alkylene group, optionally substituted C₂₋₁₀ alkenylene group, optionally substituted C₂₋₁₀ alkynylene group, or optionally substituted bivalent aromatic group) is handled in a container: in an atmosphere having a molecular oxygen concentration of 3vol% or less in the gas phase in the container; and/or in the co-presence of at least one compound selected from the group consisting of an antioxidant, N-oxyl compound, sulfur compound, amine, and Lewis acid.

2. The stabilizing method according to claim 1, **characterized in that** the antioxidant is a primary antioxidant.

3. The stabilizing method according to claim 1 or 2, **characterized in that** the handling temperature is within the range of -50°C to 85°C.

4. A stabilized carbodiimide derivative composition,
**characterized by** containing: a carbodiimide
derivative represented by general formula (1) 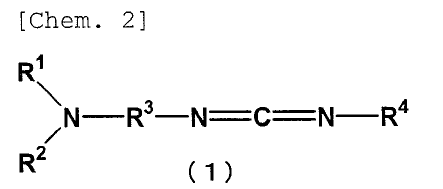 (wherein R¹, R², and R⁴ may be the same or different, and represent a hydrogen atom, an optionally substituted C₁₋₁₀ alkyl group, or optionally substituted C₇₋₁₅ aralkyl group; and R³ represents an optionally substituted C₁₋₁₀ alkylene group, optionally substituted C₂₋₁₀ alkenylene group, optionally substituted C₂₋₁₀ alkynylene group, or optionally substituted bivalent aromatic group); and
at least one compound selected from the group consisting of an antioxidant, N-oxyl compound, sulfur compound, amine, and Lewis acid.

5. The composition according to claim 4, **characterized in that** the Hazen color value is 300 units or less.

6. A packaged article, containing a carbodiimide derivative represented by general formula (1) 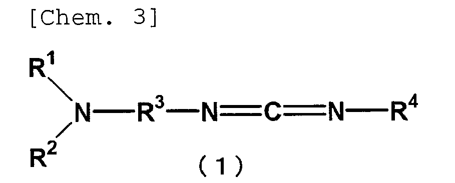 (wherein R¹, R², and R⁴ may be the same or different, and represent a hydrogen atom, an optionally substituted C₁₋₁₀ alkyl group, or optionally substituted C₇₋₁₅ aralkyl group; and R³ represents an optionally substituted C₁₋₁₀ alkylene group, optionally substituted C₂₋₁₀ alkenylene group, optionally substituted C₂₋₁₀ alkynylene group, or optionally substituted bivalent aromatic group),
**characterized by**: having a molecular oxygen concentration of 3vol% or less in the gas phase in the packaged article; and/or containing at least one compound selected from the group consisting of an antioxidant, N-oxyl compound, sulfur compound, amine, and Lewis acid.

7. The packaged article according to claim 6, **characterized in that** the volume of the packaged article is 0.5L or more.

8. The packaged article according to claim 6 or 7, **characterized in that** the packaged article contains an oxygen absorber.

9. The packaged article according to any of claims 6 to 8, **characterized in that** the Hazen color value of the carbodiimide derivative represented by general formula (1) is 300 units or less.
